# EUROPEAN PATENT SPECIFICATION

(11) **EP 2 789 289 B1**
(45) Date of publication and mention of the grant of the patent: **07.09.2016**
(21) Application number: 13846090.2
(22) Date of filing: 17.09.2013
(51) Int. Cl.: A61B 1/00, G02B 23/26

(54) **ENDOSCOPE DEVICE AND TREATMENT DEVICE**
ENDOSKOPVORRICHTUNG UND BEHANDLUNGSVORRICHTUNG
DISPOSITIF ENDOSCOPIQUE ET DISPOSITIF DE TRAITEMENT

(30) Priority: 11.10.2012 JP 2012226226
(43) Date of publication of application: 15.10.2014
(73) Proprietor: Olympus Corporation, Hachioji-shi Tokyo 192-8507 (JP)
(72) Inventor: IMAIZUMI, Katsuichi, Tokyo 192-8507 (JP); AKUI, Nobuaki, Tokyo 192-8507 (JP); ITO, Mitsuhiro, Tokyo 192-8507 (JP)
(74) Representative: Gunzelmann, Rainer
(86) International application number: PCT/JP2013/075000
(87) International publication number: WO 2014/057773

(56) References cited:
- EP-A2- 0 320 108
- JP-A- 2004 205 764
- JP-A- 2004 205 764
- JP-A- 2008 073 346
- JP-A- 2008 212 348
- JP-A- 2011 019 706
- US-A- 4 311 142
- US-A1- 2002 139 920
- US-A1- 2011 015 528

## Description

### Technical Field

The present invention relates to an endoscope apparatus and a treatment apparatus, and specifically relates to an endoscope apparatus and a treatment apparatus that detect breakage of a conductive wire for transmission of a drive signal that drives an optical fiber and control an amount of illuminating light.

### Background Art

As is well known, there are electronic endoscopes that photoelectrically convert a subject image by means of an image pickup apparatus including a solid-state image pickup device such as a CCD or CMOS and display the resulting image on a monitor. In recent years, there have been scanning endoscope apparatuses as apparatuses that display an object image without using such solid-state image pickup device technique. The scanning endoscope apparatuses cause a distal end of an illumination fiber that guides light from a light source to perform scanning, receive return light from a subject via an optical fiber bundle arranged around the illumination fiber and form an object image using light intensity signals detected over time.

For example, Japanese Patent Application Laid-Open Publication No. 2011-19706 discloses a medical observation system using a scanning medical probe that conducts laser light from a laser light source via a single-mode optical fiber to a distal end portion of an insertion portion to illuminate an object.

In the medical observation system disclosed in Japanese Patent Application Laid-Open Publication No. 2011-19706, the optical fiber is passed through a through hole in an actuator formed of, e.g., a piezoelectric element and fixed, and a drive voltage is supplied to a plurality of electrodes provided in X and Y-axis directions in the actuator to vibrate the optical fiber in a predetermined manner, thereby causing laser light to perform scanning.

In general, laser light may cause harm when, e.g., humans look directly at the laser light, and therefore, safety standards on laser light amount have been provided for apparatuses that emit laser light in, e.g., the JIS standards. Accordingly, the medical observation system disclosed in Japanese Patent Application Laid-Open Publication No. 2011-19706 determines whether or not a scanning medical probe is inserted inside a body, and based on a result of the determination, controls an amount of laser light emitted from a laser light source to control the amount of the laser light to a safe level.

However, in the medical observation system disclosed in Japanese Patent Application Laid-Open Publication No. 2011-19706, there is not any disclosure regarding control of the amount of the laser light when the optical fiber for sending the laser light from the laser light source to the object is fractured. Therefore, in the medical observation system, when the optical fiber for sending the laser light from the laser light source to the object is fractured, there has been a problem that the scanning medical probe is damaged by the laser light leaked out from a fractured part.

US 2002/139920 discloses a device according to the preamble of claims 1 and 4.

An object of the present invention is to provide an endoscope apparatus capable of preventing, even when an optical fiber for sending laser light is fractured, leakage of the laser light from a fractured part.

### Disclosure of Invention

### Means for Solving the Problem

An endoscope apparatus according to an aspect of the present invention includes: a laser light source; a light-guiding section that guides illumination light from the laser light source to an object; a conductive portion brought in close contact with the light-guiding section; a drive section provided at a distal end of the conductive portion for conducting a drive signal, the drive section swinging a distal end portion of the light-guiding section based on the drive signal; a detection section that detects that the conductive portion is broken and outputs a detection signal; and a light amount control section that controls an amount of the illuminating light based on the detection signal.

Also, a treatment apparatus according to an aspect of the present invention includes: a laser light source; a light-guiding section that guides illumination light from the laser light source to an object; a conductive portion brought in close contact with the light-guiding section; a drive section provided at a distal end of the conductive portion for conducting a drive signal, the drive section swinging a distal end portion of the light-guiding section based on the drive signal; a detection section that detects that the conductive portion is broken and outputs a detection signal; and a light amount control section that controls an amount of the illuminating light based on the detection signal.

### Brief Description of the Drawings

Fig. 1 is a diagram illustrating a configuration of an endoscope apparatus according to an embodiment;
Fig. 2 is a cross-sectional diagram for describing a configuration of a distal end portion 12 of an insertion portion 10;
Fig. 3 is a perspective diagram for describing the configuration of the distal end portion 12 of the insertion portion 10;
Fig. 4 is a flowchart illustrating an example of the flow of detection processing for detecting a fracture of an optical fiber 14; and
Fig. 5 is a diagram illustrating a configuration of an endoscope apparatus according to a modification of the present embodiment.

### Best Mode for Carrying Out the Invention

An embodiment of the present invention will be described with reference to the drawings.

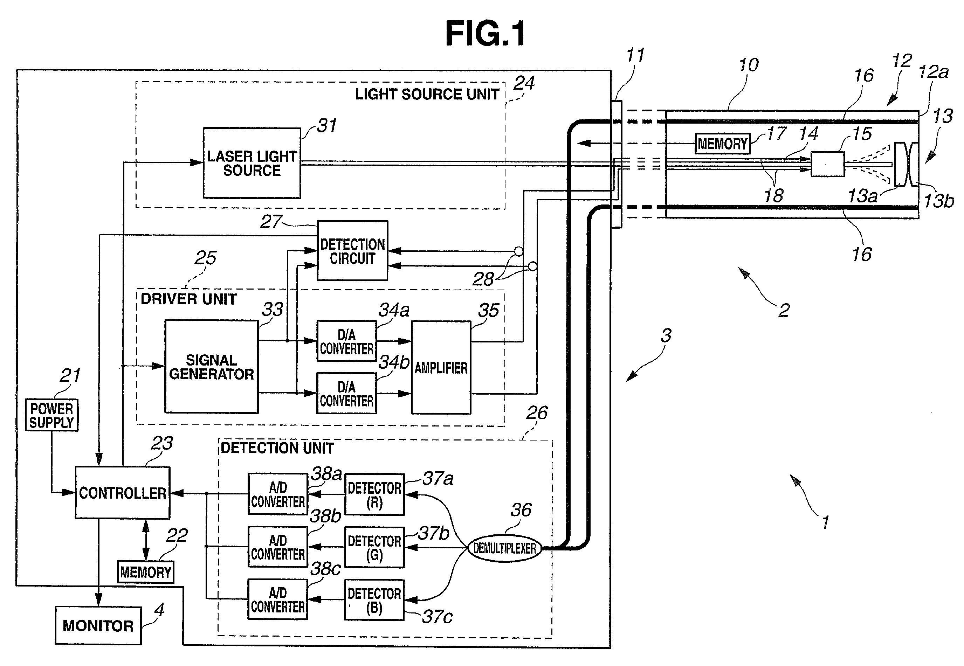

First, an overall configuration of an endoscope apparatus according to an embodiment will be described with reference to Fig. 1.

Fig. 1 is a diagram illustrating a configuration of an endoscope apparatus according to an embodiment.

As illustrated in Fig. 1, the endoscope apparatus 1 includes a scanning endoscope 2 that applies illuminating light to a subject while causing the illuminating light to perform scanning and obtains return light from the subject, a main body apparatus 3 connected to the endoscope 2, and a monitor 4 that displays a subject image obtained in the main body apparatus 3. The endoscope apparatus 1 is a treatment apparatus for treating a site of lesion using a non-illustrated treatment instrument while observing the site of lesion in a body cavity via the endoscope 2.

The endoscope 2 includes an elongated insertion portion 10 mainly including a tube body having predetermined flexibility, the insertion portion 10 being inserted inside a living body. On the proximal end side of the insertion portion 10, a connector 11 is provided, and the endoscope 2 is detachably attachable to the main body apparatus 3 via the connector 11. Also, on the distal end side of the insertion portion 10, a distal end portion 12 is provided.

At a distal end face 12a of the distal end portion 12, a distal end optical system 13 including illumination lenses 13a and 13b is provided. Also, inside the insertion portion 10, an optical fiber 14, which serves as an optical element inserted from the proximal end side to the distal end side, the optical element guiding light from a later-described light source unit 24 and applies the illuminating light to a living body, and actuators 15 provided on the distal end side of the optical fiber 14, the actuators 15 each causing a distal end of the optical fiber 14 to perform scanning in a predetermined direction based on a drive signal from a later-described driver unit 25. With such configuration, illuminating light from the light source unit 24 that has been guided via the optical fiber 14 is applied to an object.

Also, inside the insertion portion 10, detection fibers 16, which serve as a light receiving section inserted from the proximal end side to the distal end side along an inner periphery of the insertion portion 10, the light receiving section receiving return light from a subject. Distal end faces of the detection fibers 16 are arranged around the distal end optical system 13 at the distal end face 12a of the distal end portion 12. The detection fibers 16 may be a fiber bundle including at least two fibers. When the endoscope 2 is connected to the main body apparatus 3, the detection fiber 16 is connected to a later-described demultiplexer 36.

Also, inside the insertion portion 10, a memory 17 that stores various types of information relating to the endoscope 2 is provided. When the endoscope 2 is connected to the main body apparatus 3, the memory 17 is connected to a later-described controller 23 via a non-illustrated signal wire, and the various types of information relating to the endoscope 2 are read by the controller 23.

A plurality of conductive wires 18 each including, for example, a line-shaped metal are vapor-deposited on the optical fiber 14 from the connector 11 to the actuators 15 in the distal end portion 12. At distal ends of the plurality of conductive wires 18, the actuators 15 are provided. The plurality of conductive wires 18 are connected to an amplifier 35 in the main body apparatus 3 when the connector 11 is attached to the main body apparatus 3. The plurality of conductive wires 18 provide a conductive portion that conducts drive signals for driving the actuators 15.

The main body apparatus 3 includes a power supply 21, a memory 22, the controller 23, the light source unit 24, the driver unit 25, a detection unit 26, a detection circuit 27 and a current probe 28.

The light source unit 24 includes a laser light source 31. The driver unit 25 includes a signal generator 33, digital-analog (hereinafter referred to as "D/A") converters 34a and 34b and the amplifier 35.

The detection unit 26 includes the demultiplexer 36, detectors 37a to 37c and analog-digital (hereinafter referred to as "A/D") converters 38a to 38c.

The power supply 21 controls supply of power to the controller 23 in response to an operation of, e.g., a non-illustrated power supply switch. In the memory 22, e.g., a control program for performing overall control of the main body apparatus 3 is stored.

Upon supply of power from the power supply 21, the controller 23 reads the control program from the memory 22 to control the source unit 24 and the driver unit 25, and analyzes an intensity of return light from an object, which has been detected by the detection unit 26, and performs control to cause an obtained object image to perform displayed on the monitor 4. Also, although described later, the controller 23 performs control to stop emission of laser light from the laser light source 31 in the light source unit 24 based on a detection signal from the detection circuit 27.

The laser light source 31 of the light source unit 24 emits laser light (illuminating light) in a predetermined wavelength band to the optical fiber 14 under the control of the controller 23. The optical fiber 14 provides a light-guiding section that guides laser light (illuminating light) from the laser light source 31 to a target object.

The signal generator 33 in the driver unit 25 outputs a drive signal for causing (driving scanning of) the distal end of the optical fiber 14 to perform scanning in a desired direction, for example, in a spiral, based on the control performed by the controller 23. More specifically, the signal generator 33 outputs a drive signal for driving the distal end of the optical fiber 14 in a horizontal direction (X-axis direction) relative to an insertion axis of the insertion portion 10 to the D/A converter 34a and the detection circuit 27, and outputs a drive signal for driving the distal end of the optical fiber 14 in a vertical direction (Y-axis direction) relative to the insertion axis of the insertion portion 10 to the D/A converter 34b and the detection circuit 27.

Each of the D/A converters 34a and 34b converts the inputted drive signal from a digital signal to an analog signal and outputs the analog signal to the amplifier 35. The amplifier 35 amplifies the input drive signals and outputs the drive signals to the actuators 15. The drive signals outputted from the amplifier 35 are supplied to the actuators 15 via the plurality of conductive wires 18 vapor-deposited on the optical fiber 14.

A current probe 28 is arranged in each of the plurality of conductive wires 18, and the current probes 28 detect current values of the respective conductive wires 18 and output the current values to the detection circuit 27. Since the plurality of conductive wires 18 are vapor-deposited on the optical fiber 14, if the optical fiber 14 is fractured, the plurality of conductive wires 18 or any of the plurality of conductive wires 18 are broken, whereby all or any of the current values from the current probes 28 become zero and such current values are inputted to the detection circuit 27. Also, the drive signals from the signal generator 33 are also inputted to the detection circuit 27.

The detection circuit 27, which serves as a detection section, detects the current values inputted from the current probes 28 for a predetermined period of time based on voltages of the drive signals from the signal generator 33 to detect breakage of the conductive wires 18 due to a fracture of the optical fiber 14. In other words, if the current values from the current probes 28 are zero in spite of voltages of the drive signals from the signal generator 33 being generated, the detection circuit 27 detects that the conductive wires 18 are broken as a result of the optical fiber 14 being fractured.

Note that the detection circuit 27 is not limited to detecting the current values for a predetermined period of time, and may, for example, detect the current values when the voltages of the drive signals from the signal generator 33 reach a maximum value to detect breakage of the conductive wires 18 due to a fracture of the optical fiber 14. Upon detection of breakage of the plurality of conductive wires 18 or any of the plurality of conductive wires 18, the detection circuit 27 outputs a detection signal to the controller 23.

The controller 23, which serves as a light amount control section, controls an amount of laser light based on the detection signal from the detection circuit 27, more specifically, controls the laser light source 31 to stop output of the laser light. Also, if the detection signal from the detection circuit 27 is input before emission of laser light from the laser light source 31, the controller 23 displays an error message on the monitor 4.

The actuators 15, which serve as a drive section, swing the distal end (free end) of the optical fiber 14 based on the drive signals from the amplifier 35 to cause the distal end of the optical fiber 14 to perform scanning in a spiral. Consequently, light emitted from the light source unit 24 to the optical fiber 14 is sequentially applied to a subject in a spiral.

The detection fibers 16 receive return light resulting from the light being reflected by a surface region of the subject, and guides the received return light to the demultiplexer 36.

The demultiplexer 36 is, for example, a dichroic mirror, and demultiplexes the return light into predetermined wavelength bands. More specifically, the demultiplexer 36 demultiplexes the return light guided by the detection fiber 16 into return light beams in R, G and B wavelength bands, and outputs the return light beams to the detector 37a, 37b and 37c, respectively.

The detectors 37a, 37b and 37c detect intensities of return light beams in the R, G and B wavelength bands, respectively. Signals of the light intensities detected by the detectors 37a, 37b and 37c are outputted to the A/D converters 38, 38b and 38c, respectively.

The A/D converters 38a to 38c respectively convert the light intensity signals outputted from the detectors 37a to 37 from analog signals to digital signals and output the digital signals to the controller 23.

The controller 23 performs predetermined image processing on the digital signals from the A/D converters 38a to 38c to generate an object image and displays the object image on the monitor 4.

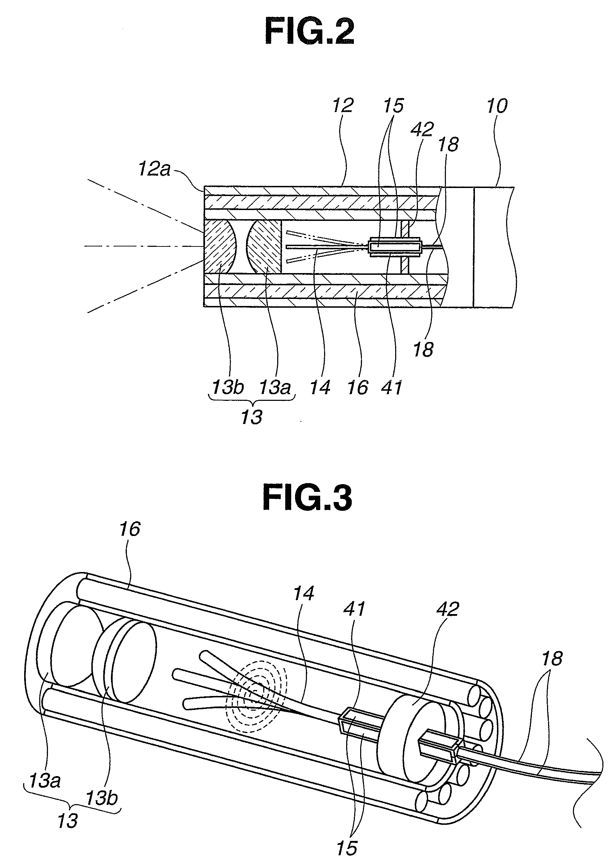

Here, a detailed configuration of the distal end portion 12 of the insertion portion 10 will be described with reference to Figs. 2 and 3.

Fig. 2 is a cross-sectional diagram for describing the configuration of the distal end portion 12 of the insertion portion 10, and Fig. 3 is a perspective diagram for describing the configuration of the distal end portion 12 of the insertion portion 10.

As illustrated in Figs. 2 and 3, in the distal end portion 12 of the insertion portion 10, a ferrule 41, which serves as a bonding member, is arranged between the optical fiber 14 and the actuator 15. The ferrule 41 is a member used in the optical communication field, and, e.g., zirconia (ceramic) or nickel is used for a material of the ferrule 41 enabling easy processing to provide a center hole with high precision (for example, ±1 µm) relative to an outer diameter of the optical fiber 14 (for example, 125 µm). At a substantial center of the ferrule 41, a through hole based on the diameter of the optical fiber 14 is provided, and the ferrule 41 is subjected to processing for provision of the center hole and the optical fiber 14 is fixed to the ferrule 41 via, e.g., an adhesive.

The ferrule 41 has a quadrangular prism shape, and the actuators 15 are arranged on respective side faces of the quadrangular prism ferrule 41. The actuators 15, the ferrule 41 and the optical fiber 14 are fixed at a substantial center of the distal end portion 12 via a fixing member 43 in the distal end portion 12.

The plurality of conductive wires 18 are deposited on the optical fiber 14. The conductive wires 18 are connected to the respective actuators 15 arranged on the respective side faces of the ferrule 41. Consequently, drive signals from the driver unit 25 are supplied to the actuators 15 via the conductive wires 18. Each of the actuators 15 is, for example, a piezoelectric element (piezo element), and expands/contracts according to a drive signal from the driver unit 25. Consequently, the distal end of the optical fiber 14 is caused to perform scanning in a spiral to apply laser light to an object.

Next, operation of the endoscope apparatus 1 configured as described above will be described.

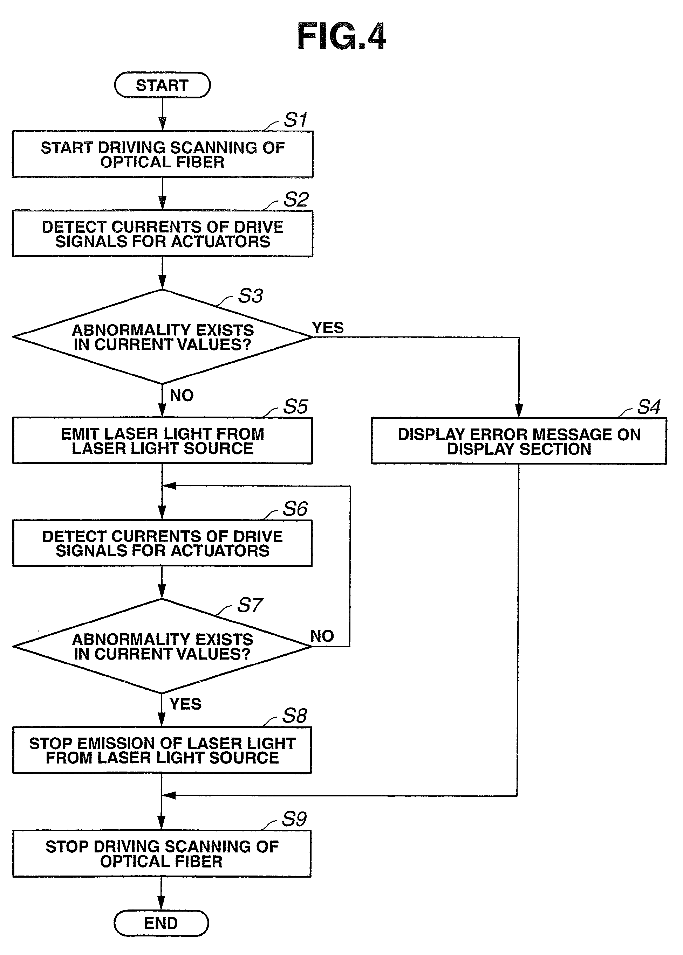

Fig. 4 is a flowchart illustrating an example of the flow of detection processing for detecting a fracture of the optical fiber 14.

First, driving of scanning of the optical fiber 14 is started based on drive signals from the signal generator 33 in the driver unit 25 (step S1). Next, currents of the drive signals for the actuators 15 are detected (step S2), and whether or not an abnormality exists in the detected current values is determined (step S3). If it is determined that an abnormality exists in the detected current values, a result of the determination is YES, an error message is displayed on the display section 4 (step S4), and the processing proceeds to step S9, which will be described later. On the other hand, if it is determined that no abnormality exists in the detected current values, the result of the determination is NO, and laser light from the laser light source 31 is emitted to the optical fiber 14 (step S5).

Next, the currents of the drive signals for the actuators 15 are detected (step S6), and whether or not an abnormality exists in the detected current values is determined (step S7). If it is determined that no abnormality exists in the current values, a result of the determination is NO, and the processing return to step S6 and processing similar to the above is repeated. On the other hand, if it is determined that an abnormality exists in the current values, the result of the determination is YES, and emission of laser light from the laser light source 31 is stopped (step S8). Lastly, the driving of scanning of the optical fiber 14 is stopped (step S9) and the processing ends.

According to the above processing, if the optical fiber 14 is fractured, the endoscope apparatus 1 can immediately stop laser light emitted from the laser light source 31 by means of the control performed by the controller 23 to which a detection signal from the detection circuit 27 is inputted, preventing leakage of the laser light from a fractured part of the optical fiber 14 and thus preventing damage of the endoscope 2.

Accordingly, even if an optical fiber that sends laser light is fractured, an endoscope apparatus according to the present embodiment enables prevention of leakage of laser light from a fractured part.

Also, in the endoscope apparatus 1, a fracture of the optical fiber 14 is detected using the conductive wires 18 that transmit drive signals to the actuators 15. Thus, the endoscope apparatus 1 does not require provision of signal wires for detection of a fracture of the optical fiber 14 and prevents the insertion portion 10 from having a large diameter compared to the conventional techniques.

### (Modification)

Next, a modification of the above-described embodiment will be described.

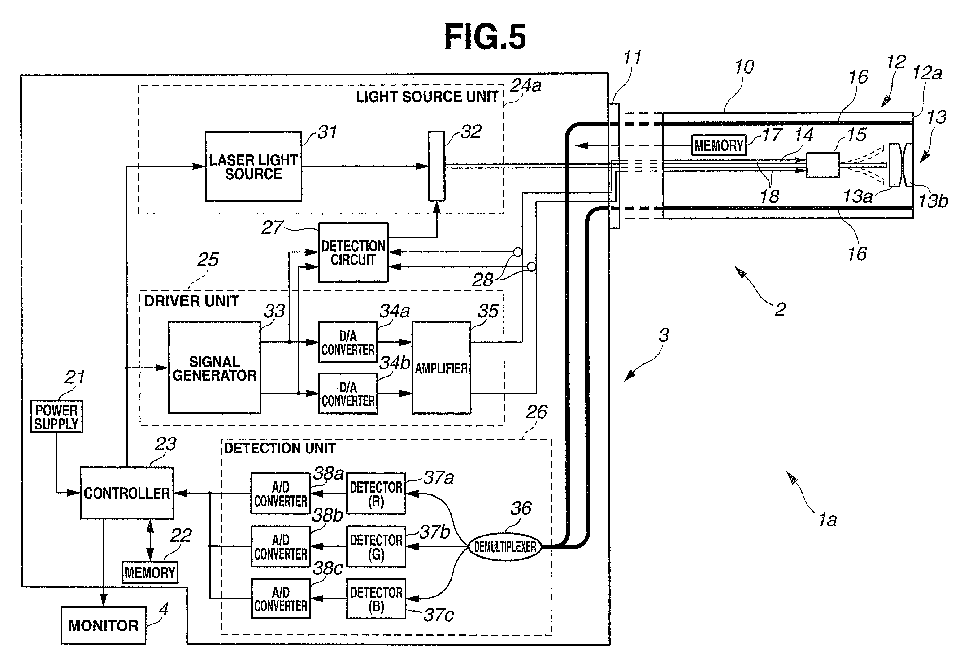

Fig. 5 is a diagram illustrating a configuration of an endoscope apparatus according to a modification of the present embodiment. In Fig. 5, components that are the same as those in Fig. 1 are provided with reference numerals that are the same as those in Fig. 1, and a description thereof will be omitted.

As illustrated in Fig. 5, an endoscope apparatus 1a includes a light source unit 24a instead of the light source unit 24 in the endoscope apparatus 1 in Fig. 1. The light source unit 24a includes a laser light source 31 and a shutter 32.

A detection signal from a detection circuit 27 is inputted to the shutter 32. Also, the shutter 32, which serves as a light amount control section, is arranged on an emission optical path of the laser light source 31, and controls an amount of laser light from the laser light source 31 based on the detection signal from the detection circuit 27 and outputs the resulting laser light to the optical fiber 14. More specifically, upon an input of a detection signal indicating that conductive wires 18 are broken from the detection circuit 27, the shutter 32 controls the amount of laser light from the laser light source 31 so that the laser light from the laser light source 31 is not outputted to the optical fiber 14. The rest of the configuration is similar to that of the above-described embodiment.

With the above configuration, if the optical fiber 14 is fractured, the endoscope apparatus 1a can prevent output of laser light emitted from the laser light source 31 to optical fiber 14 by means of control performed by the shutter 32 to which a detection signal from the detection circuit 27 is inputted, preventing leakage of the laser light from a fractured part of the optical fiber 14 and thus preventing damage of the endoscope 2.

Accordingly, as in the above-described embodiment, even if an optical fiber that sends laser light is fractured, an endoscope apparatus according to the above-described modification enables prevention of leakage of the laser light from a fractured part.

The present invention are not limited to the embodiment and the modification described above, and various modifications, alterations and the like are possible without departing from the scope of the present invention.

The present application is filed claiming the priority of Japanese Patent Application No. 2012-226226 filed in Japan on October 11, 2012.

## Claims

1. An endoscope apparatus comprising:
a laser light source (31) that generates laser light;
a light-guiding section (14) that guides the laser light inputted to a proximal end, and applies the laser light to a subject from a distal end;
a conductive portion (18) provided in close contact with the light guiding section;
a drive section (25) provided at a distal end portion of the conductive portion, the drive section swinging a distal end portion of the light-guiding section; the conductive portion being connected to the drive section;
a generation section (33) that generates a drive signal for driving the drive section, the drive signal being conducted by the conductive portion;
**characterized by**
a detection section (27) that detects a current value of a current flowing in the conductive portion when the generation section generates the drive signal, and detects that the conductive portion is broken, based on the detected current value; and
a light amount control section (23) that controls an amount of the illuminating light based on a result of detection of whether or not the conductive portion is broken in the detection section.

2. The endoscope apparatus according to claim 1, wherein the detection section detects the current value for a predetermined period of time based on a voltage of the drive signal.

3. The endoscope apparatus according to claim 2, wherein the detection section detects the current value when the voltage of the drive signal reaches a maximum value.

4. A treatment apparatus comprising:
a laser light source (31) that generates laser light;
a light-guiding section that guides the laser light inputted to a proximal end, and applies the laser light to a subject from a distal end; a conductive portion (18) provided in close contact with the light-guiding section;
a drive section provided at a distal end portion of the conductive portion, the drive section swinging a distal end portion of the light-guiding section;
the conductive portion being connected to the drive section;
a generation section (33) that generates a drive signal for driving the drive section, the drive signal being conducted by the conductive portion; **characterized by**
a detection section (27) that detects a current value of a current flowing in the conductive portion when the generation section generates the drive signal, and detects that the conductive portion is broken, based on the detected current value; and
a light amount control section (23) that controls an amount of the illuminating light based on a result of detection of whether or not the conductive portion is broken in the detection section.

5. The treatment apparatus according to claim 4, wherein the detection section detects the current value for a predetermined period of time based on a voltage of the drive signal.

6. The treatment apparatus according to claim 5, wherein the detection section detects the current value when the voltage of the drive signal reaches a maximum value.

## Patentansprüche

1. Endoskopgerät, das umfasst:
eine Laserlichtquelle (31), die Laserlicht erzeugt;
einen Lichtleitabschnitt (14), der das in ein proximales Ende eingegebene Laserlicht leitet und das Laserlicht von einem distalen Ende einem Subjekt zuführt;
einen leitfähigen Abschnitt (18), der in engem Kontakt mit dem Lichtleitabschnitt vorgesehen ist;
einen Antriebsabschnitt (25), der an einem distalen Endabschnitt des leitfähigen Abschnitts vorgesehen ist, wobei der Antriebsabschnitt einen distalen Endabschnitt des Lichtleitabschnitts schwingt;
wobei der leitfähige Abschnitt mit dem Antriebsabschnitt verbunden ist;
einen Erzeugungsabschnitt (33), der ein Antriebssignal zum Antreiben des Antriebsabschnitts erzeugt, wobei das Antriebssignal durch den leitfähigen Abschnitt geleitet wird;
**gekennzeichnet durch**
einen Erfassungsabschnitt (27), der einen Stromwert eines in dem leitfähigen Abschnitt fließenden Stroms erfasst, wenn der Erzeugungsabschnitt das Antriebssignal erzeugt, und auf der Basis des erfassten Stromwert erfasst, dass der leitfähige Abschnitt gebrochen ist; und
einen Lichtmengensteuerabschnitt (23), der auf der Basis eines Erfassungsergebnisses in dem Erfassungsabschnitt, ob der leitfähige Abschnitt gebrochen ist oder nicht, eine Menge des Beleuchtungslichts steuert.

2. Endoskopgerät nach Anspruch 1, bei dem der Erfassungsabschnitt den Stromwert für eine vorbestimmte Zeitspanne auf der Basis einer Spannung des Antriebssignals erfasst.

3. Endoskopgerät nach Anspruch 2, bei dem der Erfassungsabschnitt den Stromwert erfasst, wenn die Spannung des Antriebssignals einen Maximalwert erreicht.

4. Behandlungsgerät, das umfasst:
eine Laserlichtquelle (31), die Laserlicht erzeugt;
einen Lichtleitabschnitt (14), der das in ein proximales Ende eingegebene Laserlicht leitet und das Laserlicht von einem distalen Ende einem Subjekt zuführt;
einen leitfähigen Abschnitt (18), der in engem Kontakt mit dem Lichtleitabschnitt vorgesehen ist;
einen Antriebsabschnitt (25), der an einem distalen Endabschnitt des leitfähigen Abschnitts vorgesehen ist, wobei der Antriebsabschnitt einen distalen Endabschnitt des Lichtleitabschnitts schwingt;
wobei der leitfähige Abschnitt mit dem Antriebsabschnitt verbunden ist;
einen Erzeugungsabschnitt (33), der ein Antriebssignal zum Antreiben des Antriebsabschnitts erzeugt, wobei das Antriebssignal durch den leitfähigen Abschnitt geleitet wird;
**gekennzeichnet durch**
einen Erfassungsabschnitt (27), der einen Stromwert eines in dem leitfähigen Abschnitt fließenden Stroms erfasst, wenn der Erzeugungsabschnitt das Antriebssignal erzeugt, und auf der Basis des erfassten Stromwert erfasst, dass der leitfähige Abschnitt gebrochen ist; und
einen Lichtmengensteuerabschnitt (23), der auf der Basis eines Erfassungsergebnisses in dem Erfassungsabschnitt, ob der leitfähige Abschnitt gebrochen ist oder nicht, eine Menge des Beleuchtungslichts steuert.

5. Behandlungsgerät nach Anspruch 4, bei dem der Erfassungsabschnitt den Stromwert für eine vorbestimmte Zeitspanne auf der Basis einer Spannung des Antriebssignals erfasst.

6. Endoskopgerät nach Anspruch 5, bei dem der Erfassungsabschnitt den Stromwert erfasst, wenn die Spannung des Antriebssignals einen Maximalwert erreicht.

## Revendications

1. Appareil d'endoscope comprenant :
une source de lumière laser (31) qui génère une lumière laser ;
une section de guidage de lumière (14) qui guide la lumière laser délivrée en entrée vers une extrémité proximale, et applique la lumière laser sur un sujet depuis une extrémité distale ;
une partie conductrice (18) prévue en contact étroit avec la section de guidage de lumière ;
une section d'entraînement (25) prévue au niveau d'une partie d'extrémité distale de la partie conductrice, la section d'entraînement faisant osciller une partie d'extrémité distale de la section de guidage de lumière ;
la partie conductrice étant connectée à la section d'entraînement ;
une section de génération (33) qui génère un signal d'attaque destiné à entraîner la section d'entraînement, le signal d'attaque étant conduit par la partie conductrice ;
**caractérisé par**
une section de détection (27) qui détecte une valeur de courant d'un courant circulant dans la partie conductrice lorsque la section de génération génère le signal d'attaque, et détecte que la partie conductrice est cassée, sur la base de la valeur de courant détectée ; et
une section de commande (23) de quantité de lumière qui commande une quantité de la lumière d'éclairage sur la base d'un résultat de détection selon si oui ou non la partie conductrice est cassée dans la section de détection.

2. Appareil d'endoscope selon la revendication 1, dans lequel la section de détection détecte la valeur de courant pendant une période de temps prédéterminée sur la base d'une tension du signal d'attaque.

3. Appareil d'endoscope selon la revendication 2, dans lequel la section de détection détecte la valeur de courant lorsque la tension du signal d'attaque atteint une valeur maximale.

4. Appareil de traitement comprenant :
une source de lumière laser (31) qui génère une lumière laser ;
une section de guidage de lumière qui guide la lumière laser délivrée en entrée vers une extrémité proximale, et applique la lumière laser sur un sujet depuis une extrémité distale ;
une partie conductrice (18) prévue en contact étroit avec la section de guidage de lumière ;
une section d'entraînement prévue au niveau d'une partie d'extrémité distale de la partie conductrice, la section d'entraînement faisant osciller une partie d'extrémité distale de la section de guidage de lumière ;
la partie conductrice étant connectée à la section d'entraînement ;
une section de génération (33) qui génère un signal d'attaque destiné à entraîner la section d'entraînement, le signal d'attaque étant conduit par la partie conductrice ;
**caractérisé par**
une section de détection (27) qui détecte une valeur de courant d'un courant circulant dans la partie conductrice lorsque la section de génération génère le signal d'attaque, et détecte que la partie conductrice est cassée, sur la base de la valeur de courant détectée ; et
une section de commande (23) de quantité de lumière qui commande une quantité de la lumière d'éclairage sur la base d'un résultat de détection selon si oui ou non la partie conductrice est cassée dans la section de détection.

5. Appareil de traitement selon la revendication 4, dans lequel la section de détection détecte la valeur de courant pendant une période de temps prédéterminée sur la base d'une tension du signal d'attaque.

6. Appareil de traitement selon la revendication 5, dans lequel la section de détection détecte la valeur de courant lorsque la tension du signal d'attaque atteint une valeur maximale.
